# EUROPEAN PATENT SPECIFICATION

(11) **EP 4 408 377 B1**
(45) Date of publication and mention of the grant of the patent: **08.10.2025**
(21) Application number: 22799876.2
(22) Date of filing: 29.09.2022
(51) Int. Cl.: A61K 8/02, A61K 8/41, A61Q 5/06, A61Q 5/10

(54) **COMPOSITION COMPRISING OXIDATIVE AND DIRECT DYES**
ZUSAMMENSETZUNG MIT OXIDATIVEN UND DIREKTEN FARBSTOFFEN
COMPOSITION COMPRENANT DES COLORANTS OXYDATIFS ET DIRECTS

(30) Priority: 30.09.2021 EP 21200127
(43) Date of publication of application: 07.08.2024
(73) Proprietor: Kao Corporation, Tokyo 103-8210 (JP)
(72) Inventor: NAKAMURA, Takahito, Tokyo 131--0044 (JP); MOEHRING, Hartmut, 64297 Darmstadt (DE)
(74) Representative: Hoffmann Eitle
(86) International application number: PCT/EP2022/077125
(87) International publication number: WO 2023/052511

(56) References cited:
- EP-A1- 3 485 943
- US-A1- 2017 105 921
- US-A1- 2017 354 581

## Description

### Field of the invention

The present invention relates to composition for dyeing of keratin fibers with oxidative and direct dyes. Furthermore, a two-part or three-part composition, kit-of-parts and dyeing method is disclosed.

### Background of the invention

Direct dyes have been of particular interest of cosmetic industry over the past decade. In contrast to their oxidative counterparts, direct dyes are easier to apply to keratin fibers, but often lack durability on keratin fibers.

One of the technical challenges with direct dyes in general is their low durability on keratin fibers, which have experienced prior damage due to environmental factors or chemical services such as perming or bleaching. Chemical services alter the internal hair structure and usually allow for improved dye uptake in comparison to virgin hair, but simultaneously direct dyes are also easily washed out. Thus, consumers with prior chemical hair treatments may experience a low durability of the direct dyes, especially after several washes. Moreover, while the color intensity fades over several washes, the hair may experience an additional undesired color shift depending on the different bleeding rates of the individual direct dyes.

In everyday business operation of a hair dresser salon it is also not always easy for the professional to analyze prior hair damage of the customer, as some prior treatments may have already washed out and their effect is not visible anymore. While assuming healthy hair and then performing direct dyeing, it may come to a surprise to the hair dresser as well as the customer that durability of the dyeing treatment is exceptionally low. Such experiences will certainly frustrate every party. DE202005002552 discloses 1,4-diamino-2-methoxymethyl-benzene, oxidative dye couplers, and certain direct dyes to tackle the skin compatibility and dyeing challenges.

FR3026004 discloses 1,4-diamino-2-methoxymethyl-benzene oxidative dye couplers, and cationic direct dyes to tackle the dyeing intensity problem. US2017/354581 relates to wash fastness and employs certain direct dyes in the examples.

However, the prior art has not satisfactorily solved the above challenges, and, therefore, there is a real need to develop dyeing compositions for keratin fibers, which deliver high dyeing intensity and have improved durability, regardless of the history of chemical treatments.

### Summary of the invention

The first object of the present invention is a composition A for dyeing of keratin fibers, preferably of human keratin fibers, more preferably of human hair, comprising:
a) 1,4-diamino-2-methoxymethyl-benzene, and/or its salt(s),
b) one or more oxidative dye coupler(s) or oxidative dye precursor different from compounds according to group a),
c) one or more direct dye(s) selected from HC Blue 18, HC Red 18, and HC Yellow 16, and/or their salt(s), and/or their mixtures.

The second object of the present invention is a two-part hair dyeing composition or a three-part hair dyeing and bleaching composition comprising composition A as defined above and an aqueous composition B, preferably having a pH in the range of 1 to 6 and optionally comprising one or more oxidizing agent(s), and optionally a bleaching composition.

The third object of the present invention is a kit-of-parts comprising:
- a composition C comprising one or more compound(s) according to groups a) and b) as defined above,
- a composition D comprising one or more compound(s) according to group c) as defined above,
- an optional aqueous composition B as defined above.

The fourth object of the present invention is a method for dyeing keratin fibers, preferably human keratin fibers, more preferably human hair, comprising the steps of:
i) providing composition A as defined above and optionally mixing it with the composition B as defined above to yield a ready-to-use composition having a pH in the range of 7 to 12,
ii) applying the the ready-to-use composition onto keratin fibers and leaving it for a time period in the range of 1 min to 60 min,
iii) optionally rinsing-off the keratin fibers and optionally drying the keratin fibers.

The fifth object of the present invention is a method for dyeing keratin fibers, preferably human keratin fibers, more preferably human hair, comprising the steps of:
x) providing composition C as defined above and mixing it with composition B as defined above to prepare a ready-to-use composition having a pH in the range of 7 to 12,
xi) applying the ready-to-use composition onto keratin fibers and leaving it for a time period in the range of 1 min to 60 min, and optionally rinsing-off the keratin fibers and optionally drying the keratin fibers,
xii) applying composition D as defined above onto keratin fibers,
xiii) leaving composition D onto keratin fibers, preferably for a time period in the range of 1 min to 60 min, and optionally rinsing-off the keratin fibers, and optionally drying the keratin fibers.

### Detailed description of the invention

Inventors of the present invention have unexpectedly found out that compositions according to the independent claims improved dyeing intensity and wash fastness of direct dyes. Furthermore, the cosmetic appearance of keratin fibers was improved. The latter effects are related to shine and healthy feel.

### Composition for dyeing

The present invention is directed to a composition A for dyeing of keratin fibers, preferably of human keratin fibers, more preferably of human hair, comprising:
a) 1,4-diamino-2-methoxymethyl-benzene and/or its hydrates, and/or its salt(s),
b) one or more oxidative dye coupler(s) or oxidative dye precursor different from compounds according to group a),
c) one or more direct dye(s) selected from HC Blue 18, HC Red 18, and HC Yellow 16, and/or their salt(s), and/or their mixtures.

Suitable salts of compound(s) according to group a) is/are sulfate salt, hydrochloride salt, hydrobromide salt, nitrate salt, phosphate salt, hydrogen phosphate salt, dihydrogen phosphate salt, methosulfate salt, citrate salt, succinate salt, tartrate salt, lactate salt, tosylate salt, benzenesulfonate salt, acetate salt, and/or their mixtures, preferably it/they is/are a sulfate salt and/or a hydrochloride salt, and/or their mixtures, more preferably it is a sulfate salt, form the viewpoint of speed of dissolution.

It is preferred from the viewpoint of dyeing intensity that the total concentration of compound(s) according to group a) is 0.01% by weight or more, preferably in the 0.05% by weight or more, further more preferably 0.1% by weight or more, calculated to the total weight of composition A.

It is preferred from the viewpoint of cosmetic safety that the total concentration of compound(s) according to group a) is 60% by weight or less, preferably 50% by weight or less, further more preferably 40% by weight or less, still further more preferably 30% by weight or less, calculated to the total weight of composition A.

For attaining the above-mentioned effects, it is preferred that the total concentration of compound(s) according to group a) is in the range of 0.01% to 60% by weight, preferably in the range of 0.05% to 50% by weight, more preferably in the range of 0.1% to 40% by weight, still more preferably in the range of 0.25% to 30% by weight, calculated to the total weight of composition A.

It is preferred from the viewpoint of color shading that one or more compound(s) according to group b) is 5-amino-2-methylphenol, 2-methyl-5-hydroxyethylaminophenol, 2,4,-diaminophenoxyethanol, 2-amino-4-hydroxyethylaminoanisol, 2-methyl-5-amino-6-chlorphenol, 1,3-bis-(2,4-diaminophenoxy)-propane, 2-bis(2-hydroxyethyl)-aminotoluene, 2-amino-5-methylphenol, resorcinol, 2-methylresorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 1,3-diamino- benzene, 1-amino-3-(2'-hydroxyethylamino)benzene, 1-amino-3-[bis(2'-hydroxy-ethyl) amino]benzene, α-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)-benzene, 2,4,5,6-tetraaminopyrimidine, 2,5,6-triamino-5-pyrimidinol, and/or their salt(s), and/or their mixtures, preferably one or more compound(s) according to group b) is/are selected from 4-chlororesorcinol, 2-methylresorcinol, 1,3-bis(2,4-diaminophenoxy)-propane, 2-methyl-5-amino-6-chlorphenol, 2-amino-3-hydroxypyridine, and/or their salt(s), and/or their mixtures.

It is preferred from the viewpoint of dyeing intensity that the total concentration of compound(s) according to group b) is 0.01% by weight or more, preferably in the 0.05% by weight or more, further more preferably 0.1% by weight or more, calculated to the total weight of composition A.

It is preferred from the viewpoint of cosmetic safety that the total concentration of compound(s) according to group b) is 60% by weight or less, preferably 50% by weight or less, further more preferably 40% by weight or less, still further more preferably 30% by weight or less, calculated to the total weight of composition A.

For attaining the above-mentioned effects, it is preferred that the total concentration of compound(s) according to group b) is in the range of 0.01% to 60% by weight, preferably in the range of 0.05% to 50% by weight, more preferably in the range of 0.1% to 40% by weight, still more preferably in the range of 0.25% to 30% by weight, calculated to the total weight of composition A.

It is further preferred from the viewpoint of dyeing intensity that the total concentration of compound(s) according to groups a) and b) is 0.01% by weight or more, more preferably 0.05% by weight or more, still more preferably 0.1% by weight or more, further more preferably 0.25% by weight or more, still more preferably 0.3% by weight or more, still more preferably 0.4% by weight or more, calculated to the total weight of composition A.

It is further preferred from the viewpoint of cosmetic safety that the total concentration of compound(s) according to groups a) and b) is 60% by weight or less, preferably 50% by weight or less, more preferably 40% by weight or less, still more preferably 35% by weight or less, calculated to the total weight of composition A.

For attaining the above-mentioned effects, it is preferred that the total concentration of compound(s) according to groups a) and b) is in the range of 0.01% to 60% by weight, preferably in the range of 0.05% to 50% by weight, more preferably in the range of 0.1% to 40% by weight, still more preferably in the range of 0.25% to 35% by weight, still further more preferably in the range of 0.3% to 35% by weight, still further more preferably in the range of 0.4% to 35% by weight, calculated to the total weight of composition A.

It is preferred from the viewpoint of color shades that the weight ratio of compound(s) according to group a) to compound(s) according to group b) is 0.2 or more, more preferably 0.3 or more, still more preferably 0.5 or more.

It is preferred from the viewpoint of color shades that the weight ratio of compound(s) according to group a) to compound(s) according to group b) is 5 or less, more preferably 3 or less, still more preferably 2 or less.

For attaining the above-mentioned effects, it is preferred that the weight ratio of compound(s) according to group a) to compound(s) according to group b) is in the range of 0.2 to 5, preferably in the range of 0.3 to 3, more preferably in the range of 0.5 to 2.

It is further preferred from the viewpoint of dyeing intensity that the total concentration of one or more compound(s) according to group c) is 0.01% by weight or more, preferably 0.05% by weight or more, more preferably 0.1% by weight or more, calculated to the total weight of composition A.

It is further preferred from the viewpoint of wash fastness that the total concentration of one or more compound(s) according to group c) is 20% by weight or less, preferably 15% by weight or less, more preferably 10% by weight or less, still more preferably 5% by weight or less, calculated to the total weight of composition A.

For attaining the above-mentioned effects, it is preferred that the total concentration of one or more compound(s) according to group c) is in the range of 0.01% to 20% by weight, preferably in the range of 0.05% to 15% by weight, more preferably in the range of 0.1% to 10% by weight, more preferably in the range of 0.1% to 5% by weight, calculated to the total weight of composition A.

It is preferred from the viewpoint of dyeing intensity that the weight ratio of compound(s) according to group a) to compound(s) according to group c) is 0.1 or more, preferably 0.25 or more, more preferably 1 or more.

It is preferred from the viewpoint of wash fastness that the weight ratio of compound(s) according to group a) to compound(s) according to group c) is 20 or less, preferably 15 or less.

For attaining the above-mentioned effects, it is preferred that the weight ratio of compound(s) according to group a) to compound(s) according to group c) is in the range of 0.1 to 20, preferably in the range of 0.25 to 15, more preferably in the range of 1 to 15.

It is further preferred from the viewpoint of wash fastness that composition A of the present invention comprises one or more direct dye(s) different from group c), and/or their salt(s), and/or their mixtures. In principle, all other direct dyes are suitable to be combined. However, from the viewpoint of wash fastness it is preferred that composition A of the present invention comprises Disperse Black 9, Acid Yellow 1, 2-amino-6-chloro-4-nitrophenol, and/or their salt(s), and/or their mixtures.

Suitable total concentration of one or more compound(s) different from group c), preferably the total concentration of Disperse Black 9, Acid Yellow 1, 2-amino-6-chloro-4-nitrophenol, and/or their salt(s), and/or their mixtures, is in the range of 0.01% to 5% by weight, preferably in the range of 0.05% to 2.5% by weight, more preferably in the range of 0.1% to 1.5% by weight, calculated to the total weight of composition A.

It is preferred from the viewpoint of dyeing intensity that composition A comprises one or more alkalizing agent(s) as compound(s) according to group d), preferably one or more inorganic alkalizing agent(s), more preferably one or more metal salt of silicate, metasilicate, disilicate, hydroxide, carbonate, bicarbonate, or phosphate, more preferably one or more sodium salt(s) or potassium salt(s) of silicate, metasilicate, disilicate, hydroxide, or phosphate, and/or their mixtures, still more preferably trisodium phosphate or tripotassium phosphate, and/or their mixtures.

Further preferably, one or more compound(s) according to group d) is/are selected from sodium silicate, potassium silicate, sodium disilicate, potassium disilicate, disodium hydrogen phosphate, potassium hydrogen phosphate, trisodium phosphate, tripotassium phosphate, and/or their mixtures.

The most preferred compound(s) according to group d) is/are trisodium phosphate, tripotassium phosphate, and/or their mixtures.

It is preferred from the viewpoint of dyeing intensity that the total concentration of compound(s) according to group d), preferably the total concentration of inorganic alkalizing agents, more preferably the total concentration of one or more metal salt(s) of silicate, metasilicate, disilicate, carbonate, bicarbonate or phosphate, still more preferably the total concentration of trisodium phosphate or tripotassium phosphate, is 1% by weight or more, more preferably 5% by weight or more, further more preferably 10% by weight or more, more preferably 15% by weight or more, still more preferably 20% by weight or more, calculated to the total weight of composition A.

It is preferred from the viewpoint of cosmetic safety that the total concentration of compound(s) according to group d), preferably the total concentration of inorganic alkalizing agents, more preferably the total concentration of one or more metal salt(s) of silicate, metasilicate, disilicate, carbonate, bicarbonate, or phosphate, still more preferably the total concentration of trisodium phosphate or tripotassium phosphate, is 75% by weight or less, more preferably 70% by weight or less, further more preferably 65% by weight or less, still more preferably 60% by weight or less, still further more preferably 55% by weight or less, calculated to the total weight of composition A.

For attaining the above-mentioned effects, it is preferred that the total concentration of compound(s) according to group d), preferably the total concentration of inorganic alkalizing agents, more preferably the total concentration of one or more metal salt(s) of silicate, metasilicate, disilicate, carbonate, bicarbonate, or phosphate, still more preferably the total concentration of trisodium phosphate or tripotassium phosphate, is in the range of 1% to 75% by weight, preferably in the range of 5% to 70% by weight, more preferably in the range of 10% to 65% by weight, still more preferably in the range of 15% to 60% by weight, still further more preferably in the range of 20% to 55% by weight, calculated to the total weight of composition A.

It is preferred from the viewpoint of environmental protection that composition A may be solid composition, preferably a powder composition or a pellet composition or a tablet composition or a capsule composition, more preferably a powder hair dyeing composition or a pellet hair dyeing composition or a tablet hair dyeing composition, still more preferably it is a powder hair dyeing composition.

The term 'powder' denotes a solid composition at 25°C and atmospheric pressure. The term relates to freely flowing powders as well as compressed powders such as tablets. The powder composition may also comprise water as long as its nature of the solid state at 25°C is unchanged.

It is preferred from the viewpoint of environmental aspects that the the total concentration of water is 10% by weight or less, preferably 5% by weight or less, more preferably 1% by weight or less, still more preferably 0.1% by weight or less, calculated to the total weight of composition A, still more preferably it is an anhydrous composition.

This does not exclude the presence of residual moisture from air or crystal water bound to ingredients. Preferably, composition A is an anhydrous powder composition, from the viewpoint of stability.

It is preferred from the viewpoint of composition stability and convenience of use that composition A comprises one or more pulverulent excipient.

The term 'excipient' denotes a compound, which may act as filling material and dispersant for the other compounds of composition A and does not react with the dyes or the alkalizing agent, and, thus, confer the powder a high degree of storage stability over an extended period of time.

Composition A of the present invention may comprise an organic and/or an inorganic pulverulent excipient in which dyes are dispersed as compound(s) according to group e).

Suitable organic and/or an inorganic pulverulent excipients are, for example, diatomaceous earth, kaolin, bentonite, starch especially corn, tapioca, rice, wheat and potato, nylon powder, montmorillonite, gypsum, sawdust and perlite.

It is preferred from the viewpoint of dispersability that the total concentration of compound(s) according to group e) is 5% by weight or more, more preferably 10% by weight or more, further more preferably 15% by weight or more, still further more preferably 20% by weight or more, calculated to the total weight of composition A, from the viewpoint of achieving good dispersability of the direct dyes in the powder and quick dissolution of the powder.

It is preferred from the viewpoint of formulation freedom that the total concentration of compound(s) according to group e) is 60% by weight or less, more preferably 50% by weight or less, further more preferably 45% by weight or less, still further more preferably 40% by weight or less, calculated to the total weight of composition A, from the viewpoint of achieving good dispersability of the dyes in the powder and formulation freedom.

For attaining the above mentioned effects, it is preferred that the total concentration of compound(s) according to group e) is in the range of 5% to 60% by weight, preferably in the range of 10% to 50% by weight, further more preferably in the range of 15% to 45% by weight, still further more preferably in the range of 20% to 40% by weight, calculated to the total weight of composition A.

### Other product forms

Composition A of the present invention may be realized in other cosmetic product forms such as oil-based composition, liquid composition comprising organic solvents, or aqueous composition.

### Oil-based composition

In case composition A is an oil-based composition, it comprises one or more lipophilic compound(s) according to f) being liquid at 25°C and atmospheric pressure.

Preferably, compounds according to group f) are selected from C₇ to C₁₂ fatty alcohols, oleyl alcohol, esters of C₃ to C₂₂ alcohols with C₁₂ to C₂₂ fatty acids, C₈ to C₂₂ fatty acids, vegetable oils, and/or silicones, and/or hydrocarbon-based products, and/or their mixtures, from the viewpoint of cosmetic compatibility.

Suitable C₇ to C₁₂ fatty alcohols are 1-heptanol, 1-octanol, 1-nonanol, 1-decanol, undecyl alcohol, and lauryl alcohol, and/or their mxitures.

Suitable esters of C₃ to C₁₂ alcohols with C₁₂ to C₂₂ fatty acids are isopropyl myristate, isopropyl palmitate.

Suitable C₈ to C₂₂ fatty acids are oleic acid, linoleic acid, and palmitic acid.

Suitable vegetable oils are olive oil, almond oil, sunflower oil, and argan oil.

Suitable silicones are non-aminated and/or aminated silicones. The latter are commonly known as amodimethicones.

Suitable hydrocarbon-based products are mineral oils and paraffins, in particular light mineral oil.

It is preferred from the viewpoint of forming a stable composition and user friendliness that the total concentration of compounds according to group f) is 1% by weight or more, more preferably 5% by weight or more, further more preferably 10% by weight or more, more preferably 15% by weight or more, still more preferably 20% by weight or more, calculated to the total weight of compositions A.

It is preferred from the viewpoint of forming a stable composition that the total concentration of compounds according to group f) is 75% by weight or less, more preferably 70% by weight or less, further more preferably 65% by weight or less, more preferably 60% by weight or less, still more preferably 55% by weight or less, calculated to the total weight of compositions A.

For attaining the above-mentioned effects, it is preferred that the total concentration of compound(s) according to group f) is in the range 1% to 75% by weight, preferably in the range of 5% to 70% by weight, more preferably in the range of 10% to 65% by weight, still more preferably in the range of 15% to 60% by weight, still further more preferably in the range of 20% to 55% by weight, calculated to the total weight of composition A.

### Liquid composition comprising organic solvents

In case composition A is a liquid composition at 25°C and atmospheric pressure, it may comprise one or more organic solvent(s) as compound(s) according to group g).

It is preferred from the viewpoint of stability that the total concentration of water is 10% by weight or less, preferably 5% by weight or less, more preferably 1% by weight or less, still more preferably 0.1% by weight or less, calculated to the total weight of composition A, still more preferably it is an anhydrous composition.

The term 'anhydrous' denotes a composition A, which is free of added water. This does not exclude the presence of residual moisture from air or crystal water bound to ingredients.

For this aspect of the present invention, composition A may comprise one or more organic solvent(s).

The organic solvent(s) may be selected to dissolve the dyes. Preferred solvents are mono-, di-, and trivalent alcohols and/or their mixtures.

Preferred mono-, di-, and trivalent alcohols from the viewpoint of cosmetic safety and dissolution capacity are ethanol, n-propanol, isopropanol, propylene glycol, ethylene glycol, benzyl alcohol, phenoxyethanol, and glycerol, and/or their mixtures.

It is further preferred from the viewpoint of solution stability that the total concentration of organic solvents is 20% by weight or more, more preferably 25% by weight or more, further more preferably 30% by weight or more, calculated to the total weight of composition A.

It is further preferred from the viewpoint of dyeing intensity that the total concentration of organic solvents is 90% by weight or less, more preferably 80% by weight or less, further more preferably 75% by weight or less, calculated to the total weight of composition A.

For attaining the above-mentioned effects, it is preferred that the total concentration of organic solvents is in the range of 20% to 90% by weight, more preferably 25% to 80% by weight, further more preferably in the range of 30% to 75% by weight, calculated to the total weight of composition A.

It is preferred from the viewpoint of dyeing intensity that composition A in the present form comprises one or more alkalizing agent(s), preferably one or more organic alkalizing agent(s) and/or its/their salt(s), preferably selected from organic alkyl and/or alkanol amines and/or their salt(s) according to the following general structure: 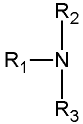 wherein R1, R2, and R3 are independently selected from H, linear C1-C6 alkyl which may be substituted with one hydroxyl group, or branched C3-C12 alkyl or alkanol, wherein at least one of R1, R2, or R3 is different from H, and/or their salts, and/or their mixtures.

Preferably, one or more organic alkalizing agent(s) are selected from alkyl and/or alkanolamine(s) and/or its/their salt(s), more preferably they/it is selected from monoethanolamine, diethanolamine, monoethanol methylamine, monoethanol dimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine, trimethylamine, triethylamine, 2-amino-2-methylpropanol, tris-(hydroxymethyl)-aminomethane and/or its/their salt(s), and/or their mixtures, from the viewpoint of providing alkalinity and cosmetic safety as well as their low odor.

The most preferred organic alkalizing agent(s) is/are selected from monoethanolamine, 2-amino-2-methylpropanol, tris-(hydroxymethyl)-aminomethane, and/or its/their salt(s), ammonia and or its salt(s), and/or their mixtures, from the viewpoint of providing alkalinity and cosmetic safety.

It is preferred from the viewpoint of dyeing intensity that the total concentration of organic alkalizing agent(s) is 0.1% by weight or more, more preferably 0.25% by weight or more, further more preferably 0.4% by weight or more, still further more preferably 1% by weight or more, still further more preferably 2% by weight or more, calculated to the total weight of composition A.

It is preferred from the viewpoint of cosmetic safety that the total concentration of organic alkalizing agent(s) is 40% by weight or less, more preferably 30% by weight or less, further more preferably 25% by weight or less, still further more preferably 20% by weight or less, still further more preferably 10% by weight or less, calculated to the total weight of composition A.

For attaining the above-mentioned effects, it is preferred that the total concentration of organic alkalizing agent(s) is in the range of 0.1% to 40% by weight, preferably in the range of 0.25% to 30% by weight, more preferably in the range of 0.4% to 25% by weight, still more preferably in the range of 1% to 20% by weight, still further more preferably in the range of 2% to 10% by weight, calculated to the total weight of composition A.

### Aqueous composition

It is preferred from the viewpoint of user convenience that composition A of the present invention is an aqueous composition.

The term 'aqueous' denotes a composition A that comprises a majority of water, i.e., composition A preferably comprises water at 30% by weight or more, further more preferably at 40% by weight or more, still more preferably at 50% by weight or more, still further more preferably at 60% by weight or more, calculated to the total weight of composition A, from the viewpoint of achieving a cosmetically acceptable composition.

It is further preferred from the viewpoint of user convenience that composition A comprises water at 99% by weight or less, more preferably at 98% by weight or less, calculated to the total weight of composition A.

For achieving the above-mentioned effects, it is preferred that the total concentration of water in composition A is in the range of 30% to 99% by weight, more preferably in the range of 40% to 98% by weight, further more preferably in the range of 50% to 98% by weight, still more preferably in the range of 60% to 98% by weight, calculated to the total weight of composition A.

It is preferred from the viewpoint of dyeing performance that the pH of composition A is 7 or more, more preferably the pH is 7.5 or more, further more preferably the pH is 8 or more, still further more preferably the pH is 9 or more.

It is preferred from the viewpoint of hair damage and dyeing performance that the pH of composition A is 12 or less, more preferably the pH is 11 or less, still more preferably the pH is 10.5 or less.

For attaining the above mentioned effects, it is preferred that the pH of composition A is in the range of 7 to 12, preferably in the range of 8 to 11.5, further more preferably in the range of 9 to 11.5, further more preferably in the range of 9 to 10.5.

The pH of the compositions is measured with a glass electrode at 25°C and under atmospheric conditions.

The skilled person recognizes that the disclosure above for composition A also refers to the ready-to-use composition.

It is preferred from the viewpoint of dyeing intensity that the total concentration of compound(s) according to group a) is 0.01% by weight or more, preferably in the 0.05% by weight or more, further more preferably 0.1% by weight or more, calculated to the total weight of composition A.

It is preferred from the viewpoint of cosmetic safety that the total concentration of compound(s) according to group a) is 20% by weight or less, preferably 15% by weight or less, further more preferably 10% by weight or less, still further more preferably 8% by weight or less, calculated to the total weight of composition A.

For attaining the above-mentioned effects, it is preferred that the total concentration of compound(s) according to group a) is in the range of 0.01% to 20% by weight, preferably in the range of 0.05% to 15% by weight, more preferably in the range of 0.1% to 10% by weight, still more preferably in the range of 0.1% to 8% by weight, calculated to the total weight of composition A.

It is preferred from the viewpoint of dyeing intensity that the total concentration of compound(s) according to group b) is 0.01% by weight or more, preferably in the 0.05% by weight or more, further more preferably 0.1% by weight or more, calculated to the total weight of composition A.

It is preferred from the viewpoint of cosmetic safety that the total concentration of compound(s) according to group b) is 20% by weight or less, preferably 15% by weight or less, further more preferably 10% by weight or less, still further more preferably 8% by weight or less, calculated to the total weight of composition A.

For attaining the above-mentioned effects, it is preferred that the total concentration of compound(s) according to group b) is in the range of 0.01% to 20% by weight, preferably in the range of 0.05% to 15% by weight, more preferably in the range of 0.1% to 10% by weight, still more preferably in the range of 0.1% to 8% by weight, calculated to the total weight of composition A.

It is preferred from the viewpoint of dyeing intensity that the total concentration of compound(s) according to groups a) and b) is 0.01% by weight or more, preferably in the 0.05% by weight or more, further more preferably 0.1% by weight or more, further more preferably 0.25% by weight or more, calculated to the total weight of composition A.

It is preferred from the viewpoint of cosmetic safety that the total concentration of compound(s) according to groups a) and b) is 20% by weight or less, preferably 15% by weight or less, further more preferably 10% by weight or less, still further more preferably 8% by weight or less, calculated to the total weight of composition A.

For attaining the above-mentioned effects, it is preferred that the total concentration of compound(s) according to groups a) and b) is in the range of 0.01% to 20% by weight, preferably in the range of 0.05% to 15% by weight, more preferably in the range of 0.1% to 12% by weight, still more preferably in the range of 0.25% to 10% by weight, still further more preferably in the range of 0.25% to 8% by weight, calculated to the total weight of composition A.

It is preferred from the viewpoint of dyeing intensity that the total concentration of compound(s) according to group c) is 0.01% by weight or more, preferably in the 0.05% by weight or more, further more preferably 0.1% by weight or more, calculated to the total weight of composition A.

It is preferred from the viewpoint of cosmetic safety that the total concentration of compound(s) according to group c) is 5% by weight or less, preferably 2.5% by weight or less, further more preferably 1.5% by weight or less, still further more preferably 8% by weight or less, calculated to the total weight of composition A.

For attaining the above-mentioned effects, it is preferred that the total concentration of compound(s) according to group c) is in the range of 0.01% to 5% by weight, preferably in the range of 0.05% to 2.5% by weight, more preferably in the range of 0.1% to 1.5% by weight, calculated to the total weight of composition A.

It is preferred from the viewpoint of product safety, dyeing intensity, and hair feel that composition A comprises one or more lipophilic compound(s) according to group f).

Lipophilic compounds within the meaning of the present invention are liquid compounds at 25°C and atmospheric pressure, which do not fully mix with water at 25°C under atmospheric pressure.

It is preferred that composition A comprises one or more lipophilic compound(s) according to group f) at a total concentration in the range of 0.1% to 15% by weight, more preferably in the range of 0.25% to 12% by weight, still more preferably in the range of 0.5% to 10% by weight, calculated to the total weight of composition A

It is preferred from the viewpoint of dyeing intensity that composition A comprises one or more alkalizing agent(s), preferably one or more organic alkalizing agent(s) and/or its/their salt(s), preferably selected from organic alkyl and/or alkanol amines and/or their salt(s) according to the following general structure: 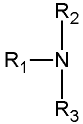 wherein R1, R2, and R3 are independently selected from H, linear C1-C6 alkyl which may be substituted with one hydroxyl group, or branched C3-C12 alkyl or alkanol, wherein at least one of R1, R2, or R3 is different from H, and/or their salts, and/or their mixtures.

Preferred organic alkalizing agent(s) is/are selected from alkyl and/or alkanolamine(s) and/or its/their salt(s), more preferably they/it is selected from monoethanolamine, diethanolamine, monoethanol methylamine, monoethanol dimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine, trimethylamine, triethylamine, 2-amino-2-methylpropanol, tris-(hydroxymethyl)-aminomethane and/or its/their salt(s), and/or their mixtures.

It is preferred from the viewpoint of dyeing intensity that the total concentration of one or more alkalizing agent(s) in composition A is 0.1% by weight or more, more preferably 0.25% by weight or more, still more preferably 0.4% by weight or more, still more preferably 1% by weight or more, still further more preferably 2% by weight or more, calculated to the total weight of composition A.

It is preferred from the viewpoint of cosmetic safety that the total concentration of one or more alkalizing agent(s) in composition A is 40% by weight or less, more preferably 30% by weight or less, still more preferably 25% by weight or less, still more preferably 20% by weight or less, still further more preferably 10% by weight or less, calculated to the total weight of composition A.

For attaining the above-mentioned effects, it is preferred that the total concentration of alkalizing agents in composition A is in the range of 0.1% to 40% by weight, preferably in the range of 0.25% to 30% by weight, more preferably in the range of 0.4% to 25% by weight, still more preferably in the range of 1% to 20% by weight, still further more preferably in the range of 2% to 10% by weight, calculated to the total weight of composition A.

### Surfactants as compounds according to g)

Compositions A, B, C, and/or D of the present invention may further comprise one or more surfactant(s) as compound according to group g), preferably selected from non-ionic surfactants, anionic surfactants, cationic surfactants, and/or amphoteric/zwitterionic surfactants, and/or their salt(s), and/or their mixtures, more preferably selected from anionic surfactants and/or their salt(s), from the viewpoint of stabilizing the composition and improving wettability and mixability.

Preferably, the anionic surfactants may be selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures, and/or their salts.

Suitable examples are alkyl sulfate or preferably ethoxylated alkyl ether sulfate surfactants or mixtures thereof, and/or salts thereof, having an alkyl chain length of C₁₀ to C₂₂ and an ethoxylation degree from 1 to 50.

Suitable non-ionic surfactants may be selected from alkyl polyglycosides, ethoxylated triglycerides, ethoxylated fatty alcohols, ethoxylated fatty acid esters, and/or their mixtures.

Suitable cationic surfactants are quaternary ammonium surfactants having a carbon chain length in the range of C₁₂ to C₂₂ or surfactants having a tertiary amine group and at least one alkyl chain having a carbon chain length in the range of C₁₂ to C₂₂ such as alkylamidoalkylamine surfactants, and/or their salts. Suitable examples are cetrimonium chloride and behentrimonium chloride.

Suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

Suitable concentration ranges for surfactants are in the range of 0.1% to 10% by weight, calculated to the total weight of each of the compositions A, B, C, and/or D, from the viewpoint of enhancing wettability of keratin fibers, physical stability, and mixability with other compositions.

### Thickening polymers

From the viewpoint of cosmetic safety, it is further preferred that the compositions A, B, C, and/or D comprise one or more thickening polymer.

Composition A, B, C, and/or D may comprise one or more thickening polymer(s) selected from non-ionic thickening polymers and/or anionic thickening polymers, and/or their mixtures.

Preferably, the thickening polymers are selected from polymers resulting in an aqueous solution and/or aqueous dispersion at pH ranges between 7 and 12 having a viscosity of at least 1,000 mPa•s measured at a polymer concentration of 1% by weight in water at 25°C determined by cone plate viscometry at 25°C under atmospheric conditions, calculated to the total weight of the composition, determined by cone-plate viscometry, suitably with a Brookfield viscometer with spindle #4 measured at 10 rpm for 1 min at 25°C and atmospheric conditions.

Suitable non-ionic thickening polymers are cellulose-based polymers. Suitable examples of cellulose-based polymers are methylcellulose, hydroxymethylcellulose, hydroxyethylcellulose, hydroxypropylcellulose, hydroxyethyl-methylcellulose, and alkylated hydroxyl celluloses such as (C₂-C₈)-alkylcelluloses or cetyl hydroxyethylcellulose.

Suitable anionic thickening polymers are selected from naturally-based anionic polymers and/or synthetic anionic polymers.

Suitably, the natural anionic polymer(s) may be selected from xanthan gum, dehydroxanthan gum, hydroxypropylxanthan gum, carboxymethyl cellulose and starch based polymers such as vegetable starch and/or their synthetically modified derivatives such as hydroxypropyl starch phosphate. Equally suitable are alginic acids, sodium alginates, ammonium alginates, calcium alginates, gum arabic, and guar gum.

Suitable synthetic anionic polymers are associative thickening polymers, such as acrylates/steareth-30 methacrylate copolymer.

The preferred thickening polymer for the composition of the present invention are natural anionic polymers, more preferably xanthan gum and/or dehydroxanthan gum, from the viewpoint of their biodegradability and low environmental impact.

Preferably, the total concentration of thickening polymers of the present invention are 0.1% by weight or more, more preferably 0.25% by weight or more, more preferably 0.5% by weight or more, calculated to the total weight of each of the compositions A, B, C, and/or D, from the viewpoint of providing sufficient viscosity to the composition.

Preferably, the total concentration of thickening polymers of the present invention are 15% by weight or less, more preferably 12% by weight or more, further more preferably 10% by weight or less, calculated to the total weight of each of the compositions A, B, C, and/or D, from the viewpoint of providing sufficient viscosity to the composition and cost of goods.

For attaining the above-mentioned effects, it is preferred that the total concentration of thickening polymers in the composition of the present invention is in the range of 0.1% to 15% by weight, preferably in the range of 0.25% to 12% by weight, more preferably in the range of 0.5% to 10% by weight, calculated to the total weight of each of the compositions A, B, C, and/or D.

It is preferred from the viewpoint of cosmetic safety that the composition of the present invention, in case that it is aqueous, has a viscosity in the range of 1,000 Pas to 25,000 mPas, preferably 2,000 mPas to 20,000 mPas, more preferably in the range of 2,500 mPas to 17,500 mPas, determined by cone plate viscometry at 25°C under atmospheric conditions. A method for determining the viscometry is cone-plate viscometry, suitably with a Brookfield viscometer and spindle #4, measured at 10 rpm for 1 min at 25°C.

### Oxidizing agents

Compositions A and/or B may comprise one or more oxidizing agent(s), preferably hydrogen peroxide.

It is further preferred from the viewpoint of dyeing performance that the concentration of hydrogen peroxide in the composition is 0.1% by weight or more, more preferably 0.25% by weight or more, further more preferably 1% by weight or more, calculated to the total weight of each of the compositions A and/or B.

It is further preferred from the viewpoint of product performance and user safety that the concentration of hydrogen peroxide in the composition is 20% by weight or less, more preferably 15% by weight or less, further more preferably 12% by weight or less, calculated to the total weight of each of the compositions A and/or B.

For attaining the above-mentioned effects, it is preferred that the concentration of hydrogen peroxide in the composition is in the range of 0.1% to 20% by weight, more preferably in the range of 0.25% to 15% by weight, further more preferably in the range of 1% to 12% by weight, calculated to the total weight of each of the compositions A and/or B.

### Two-part or three-part composition

The present invention is also directed to a two-part hair dyeing composition or a three-part hair dyeing and bleaching composition comprising composition A as defined above and an aqueous composition B, preferably having a pH in the range of 1 to 6 and optionally comprising one or more oxidizing agent(s), and optionally a bleaching composition.

In case composition A of the present invention as defined above already comprises one or more oxidizing agents, the composition B may be free of oxidizing agents.

In case composition A does not comprise oxidizing agents, it is preferred from the viewpoint of dyeing intensity that the composition B has a pH in the range of 1 to 6 and comprises one or more oxidizing agent(s), preferably hydrogen peroxide.

Preferably, from the viewpoint of stability, the composition B has a pH in the range of 1.5 to 5, further more preferably in the range of 2 to 4.5

Composition B preferably is an emulsion, thickened gel, or a combination thereof, from the viewpoint of cosmetic safety as well as user friendliness. It may comprise lipophilic compound(s) according to group f) and/or surfactant(s) according to group g) and/or one or more thickening polymer(s) as defined above.

Composition B may further comprise one or more oxidizing agent(s). The preferred oxidizing agent is hydrogen peroxide.

Suitable concentration ranges for hydrogen peroxide in composition B is/are in the range of 0.1% to 20% by weight, more preferably in the range of 0.25% to 15% by weight, further more preferably in the range of 1% to 12% by weight, calculated to the total weight of the composition B.

Composition B may comprise one or more lipophilic compound(s) as compound(s) according to group f), as defined for the composition of the present invention above.

It is preferred from the viewpoint of forming a stable composition and user friendliness that the total concentration of compounds according to group f) is 1% by weight or more, more preferably 2% by weight or more, further more preferably 3% by weight or more, calculated to the total weight of composition B.

It is preferred from the viewpoint of forming a stable composition that the total concentration of compounds according to group f) is 20% by weight or less, more preferably 15% by weight or less, further more preferably 12% by weight or less, calculated to the total weight of composition B.

For attaining the above-mentioned effects, the total concentration of compounds according to group f) is in the range of 1% to 20% by weight, preferably in the range of 2% to 15% by weight, more preferably in the range of 3% to 12% by weight, calculated to the total weight of composition B.

Composition B may further comprise one or more surfactant(s) as compound according to group g).

Suitable concentration ranges for surfactants are in the range of 0.1% to 10% by weight, calculated to the total weight of composition B, from the viewpoint of enhancing wettability of keratin fibers, physical stability, and mixability with other compositions.

From the viewpoint of cosmetic safety, it is further preferred that composition B comprises one or more thickening polymer, as defined for the composition of the present invention above.

Preferably, the total concentration of thickening polymers in composition B is 0.1% by weight or more, more preferably 0.25% by weight or more, more preferably 0.5% by weight or more, calculated to the total weight of composition B, from the viewpoint of providing sufficient viscosity to composition B.

Preferably, the total concentration of thickening polymers in composition B is 15% by weight or less, more preferably 12% by weight or more, further more preferably 10% by weight or less, calculated to the total weight of composition B, from the viewpoint of providing sufficient viscosity to composition B and cost of goods.

For attaining the above-mentioned effects, it is preferred that the total concentration of thickening polymers in composition B is in the range of 0.1% to 15% by weight, preferably in the range of 0.25% to 12% by weight, more preferably in the range of 0.5% to 10% by weight, calculated to the total weight of composition B.

It is preferred from the viewpoint of cosmetic safety that composition B has a viscosity in the range of 1,000 Pas to 25,000 mPas, preferably 2,000 mPas to 20,000 mPas, more preferably in the range of 2,500 mPas to 17,500 mPas, determined by cone plate viscometry at 25°C under atmospheric conditions, suitably with a Brookfield viscometer and spindle #4, measured at 10 rpm for 1 min at 25°C.

In case the composition is a three-part hair dyeing and bleaching composition, it comprises a bleaching composition as third composition. The bleaching composition comprises one or more bleaching compound(s), preferably it comprises one or more persalt(s) and/or peroxy salt(s), more preferably in a total concentration of 1% to 80% by weight, calculated to the total weight of the bleaching composition.

### Kit-of-parts

The present invention is also directed to a kit-of-parts comprising:
- a composition C comprising one or more compound(s) according to groups a) and b) as defined above,
- a composition D comprising one or more compound(s) according to group c) as defined above, preferably having a pH in the range of 3 to 12,
- an optional composition B as defined above.

For compositions C and D the same disclosure applies as for composition A, as defined above.

Composition C preferably comprises one or more alkalizing agent(s), as defined for composition A.

### Method for dyeing

The present invention is also directed to a method for dyeing keratin fibers, preferably human keratin fibers, more preferably human hair, comprising the steps of:
i) providing composition A as defined above and optionally mixing it with the composition B as defined above to yield a ready-to-use composition having a pH in the range of 7 to 12,
ii) applying the composition A or the ready-to-use composition onto keratin fibers and leaving it for a time period in the range of 1 min to 60 min,
iii) optionally rinsing-off the keratin fibers and optionally drying the keratin fibers.

If composition A comprises oxidizing agents, then composition B may be free of oxidizing agents.

The ready-to-use composition is applied to keratin fibers and preferably left for a time period of 1 min to 60 min as defined in step ii). Further preferred time ranges for step ii) are 5 min to 45 min, more preferred ranges are 10 min to 35 min, from the viewpoint of sufficiently dyeing.

Optionally, heat may be applied while leaving the composition A or the ready-to-use composition onto keratin fibers. Suitable temperature ranges are 30°C to 50°C.

The present invention is also directed to a method for dyeing keratin fibers, preferably human keratin fibers, more preferably human hair, comprising the steps of:
x) applying composition C onto keratin fibers as defined above having a pH in the range of 7 to 12,
xi) leaving composition C of step x) onto keratin fibers for a time period in the range of 1 min to 60 min, and optionally rinsing-off the keratin fibers and optionally drying the keratin fibers,
xii) applying composition D onto keratin fibers as defined above having a pH in the range of 3 to 12,
xiii) optionally leaving composition D of step xii) onto keratin fibers for a time period in the range of 1 min to 60 min, optionally rinsing-off the keratin fibers and optionally drying the keratin fibers.

The time difference between steps xi) and xii) may be up to 72 h. It is preferred from the viewpoint of dyeing intensity that steps xi) and xii) are applied subsequently. Composition D may be a leave-in composition. Thus, step xiii) is optional.

The following examples are to illustrate the invention, but not to limit it.

### EXAMPLES

**Table 1**

| **Ingredients** | **Inv. Ex. 1** | **Inv. Ex. 2** | **Inv. Ex. 3** | **Inv. Ex. 4** | **Comp. Ex. 1** | **Comp. Ex. 2** | **Comp. Ex. 3** | **Comp. Ex. 4** |
|---|---|---|---|---|---|---|---|---|
| | **[% by weight]** | | | | | | | |
| 1,4-diamino-2-methoxymethyl-benzene | 0.18 | 0.063 | 0.097 | 0.243 | 0.18 | 0.063 | 0.097 | 0.243 |
| 4-Chlororesorcinol | 0.17 | - | - | - | 0.17 | - | - | - |
| 1,3-Bis(2,4-diamino-phenoxy)-propane 4HCI | - | 0.18 | - | - | - | 0.18 | - | - |
| 5-Amino-6-chloro-o-cresol | - | - | - | 0.25 | - | - | - | 0.25 |
| 2-Amino-3-hydroxypyridine | - | - | 0.10 | - | - | - | 0.10 | - |
| HC Yellow 16 | 0.20 | 0.20 | 0.20 | 0.20 | - | - | - | - |
| HC Yellow 4 | - | - | - | - | 0.20 | 0.20 | 0.20 | 0.20 |
| Monoethanolamine | 2.50 | | | | | | | |
| Sodium sulphite | 0.25 | | | | | | | |
| Water | Ad 100.0 | | | | | | | |
| Wash fastness (20x) | | | | | | | | |
| Δ b* | 2 | 1 | 2 | 1 | 4 | 11 | 14 | 8 |

The inventive compositions showed improved wash fastness and changes of b-values closer to 0, whereas the comparative compositions showed stronger b-shift, indicating higher color loss of the yellow dyes.

**Table 2**

| **Ingredients** | **Inv. Ex. 5** | **Inv. Ex. 6** | **Inv. Ex. 7** | **Comp. Ex. 5** | **Comp. Ex. 6** | **Comp. Ex. 7** |
|---|---|---|---|---|---|---|
| | **[% by weight]** | | | | | |
| 1,4-diamino-2-methoxymethyl-benzene | 0.45 | 0.243 | 0.139 | 0.45 | 0.243 | 0.139 |
| 4-Chlororesorcinol | 0.37 | - | - | 0.37 | - | - |
| 1,3-Bis(2,4-diamino-phenoxy)-propane 4HCI | - | 0.25 | - | - | 0.25 | - |
| 5-Amino-6-chloro-o-cresol | - | - | - | - | - | - |
| 2-Amino-3-hydroxypyridine | - | - | 0.10 | - | - | 0.10 |
| HC Blue 18 | 0.20 | 0.20 | 0.20 | - | - | - |
| HC Blue 11 | - | - | - | 0.20 | 0.20 | 0.20 |
| Monoethanolamine | 2.50 | | | | | |
| Sodium sulphite | 0.25 | | | | | |
| Water | Ad 100.0 | | | | | |
| Wash fastness (20x) | | | | | | |
| Δ b* | 1 | 1 | 0 | 7 | 6 | 11 |

The inventive compositions showed improved wash fastness and changes of b-values closer to 0, whereas the comparative compositions showed stronger b-shift, indicating higher color loss of the blue dyes.

**Table 3**

| **Ingredients** | **Inv. Ex. 8** | **Comp. Ex. 8** | **Comp. Ex. 9** |
|---|---|---|---|
| | **[% by weight]** | | |
| 1,4-diamino-2-methoxymethyl-benzene | 0.097 | 0.097 | 0.097 |
| 5-Amino-6-chloro-o-cresol | 0.1 | 0.1 | 0.1 |
| HC Red 18 | 0.20 | - | - |
| HC Red 3 | - | 0.2 | - |
| Acid Red 52 | - | - | 0.2 |
| Monoethanolamine | 2.50 | | |
| Sodium sulphite | 0.25 | | |
| Water | Ad 100.0 | | |
| Wash fastness (20x) | | | |
| Δ a* | 0 | 6 | 5 |

The inventive composition showed improved wash fastness and changes of a-value of 0, whereas the comparative compositions showed stronger a-shift, indicating higher color loss of the red dyes.

### Methods

### Hair dyeing

Goat hairstreaks (21 cm, 2 g per bundle) were permed with a commercial perming product available under the trade name Goldwell Topform Fix Concentrate for 20 min, rinsed, and then treated with an oxidative composition for 10 min for perm fixing. The hair streaks were shampooed and dried.

The dyeing compositions of tables 1-3 were freshly prepared and mixed with an oxidative composition comprising 6% by weight of hydrogen peroxide in a weight ratio of 1:1 to prepare ready-to-use compositions having a pH in the range of 10.0 to 10.5.

2 g of the ready-to-use compositions were applied onto the permed goat hairstreaks for 30 min at 30°C. The streaks were then rinsed off and dried. The colormetric data were obtained with a color-difference meter (Datacolor Check II Plus) in the CIE colorimetric system (a*,b*).

### Wash fastness

To the dyed hair streaks 1 g of commercial shampoo was applied available under the trade name Goldwell Dualsenses Color Fade Stop Shampoo and it was foamed up with lukewarm water for 30 s. The hair streaks were then rinsed off. These steps were repeated for 20 times. After treatment, the hairstreaks were blow-dried and the remaining color was measured (a*,b*). Δa* and Δb* were calculated as the color difference between 20 times washed and freshly colored hair.

The following examples are within the scope of the present invention.

### Inventive Example 9

### Composition A

| | **% by weight** |
|---|---|
| 1,4-diamino-2-methoxymethyl-benzene | 0.35 |
| 5-Amino-6-chloro-o-cresol | 0.5 |
| HC Blue 18 | 0.05 |
| HC Red 18 | 0.08 |
| HC Yellow 16 | 0.02 |
| Ammonium sulfate | 10.0 |
| Diatomaceous earth | ad 100.0 |

Composition A is mixed with an oxidative composition comprising 6% by weight of hydrogen peroxide in a weight ratio of 1:1 to prepare ready-to-use compositions having a pH in the range of 10.0 to 10.5.

### Inventive Example 10

### Composition A

| | **% by weight** |
|---|---|
| 1,4-diamino-2-methoxymethyl-benzene | 10.0 |
| 5-Amino-6-chloro-o-cresol | 9.0 |
| HC Blue 18 | 0.5 |
| HC Red 18 | 0.5 |
| HC Yellow 16 | 0.5 |
| Ammonium sulfate | 10.0 |
| Diatomaceous earth | ad 100.0 |

Composition A is mixed with an oxidative composition comprising 6% by weight of hydrogen peroxide in a weight ratio of 1:1 to prepare ready-to-use compositions having a pH in the range of 10.0 to 10.5.

### Inventive Example 11

### Composition A

| | **% by weight** |
|---|---|
| 1,4-diamino-2-methoxymethyl-benzene | 0.097 |
| 5-Amino-6-chloro-o-cresol | 0.1 |
| HC Blue 18 | 0.05 |
| HC Red 18 | 0.08 |
| HC Yellow 16 | 0.02 |
| 2-amino-2-methylpropanol | 10.0 |
| 1,2-propylene glycol | ad 100.0 |

Composition A is mixed with an oxidative composition comprising 6% by weight of hydrogen peroxide in a weight ratio of 1:1 to prepare ready-to-use compositions having a pH in the range of 10.0 to 10.5.

## Claims

1. A composition A for dyeing of keratin fibers, preferably human keratin fibers, more preferably human hair, comprising:
a) 1,4-diamino-2-methoxymethyl-benzene, and/or its salt(s),
b) one or more oxidative dye coupler(s) or oxidative dye precursor(s) different from compounds according to group a),
c) one or more direct dye(s) selected from HC Blue 18, HC Red 18, and HC Yellow 16, and/or their salt(s), and/or their mixtures.

2. The composition according to claim 1 **characterized in that** one or more compound(s) according to group b) is 5-amino-2-methylphenol, 2-methyl-5-hydroxyethylaminophenol, 2,4,-diaminophenoxyethanol, 2-amino-4-hydroxyethylaminoanisol, 2-methyl-5-amino-6-chlorphenol, 1,3-bis-(2,4-diaminophenoxy)-propane, 2-bis(2-hydroxyethyl)-aminotoluene, 2-amino-5-methylphenol, resorcinol, 2-methylresorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 1,3-diamino- benzene, 1-amino-3-(2'-hydroxyethylamino)benzene, 1-amino-3-[bis(2'-hydroxy-ethyl) amino]benzene, α-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)-benzene, 2,4,5,6-tetraaminopyrimidine, 2,5,6-triamino-5-pyrimidinol, and/or their salt(s), and/or their mixtures, preferably one or more compound(s) according to group b) is/are selected from 4-chlororesorcinol, 2-methylresorcinol, 1,3-bis(2,4-diaminophenoxy)-propane, 2-methyl-5-amino-6-chlorphenol, 2-amino-3-hydroxypyridine, and/or their salt(s), and/or their mixtures.

3. The composition according to any of the preceding claims **characterized in that** composition A is a solid composition, preferably a powder composition or a pellet composition or a tablet composition or a capsule composition, more preferably a powder hair dyeing composition or a pellet hair dyeing composition or a tablet hair dyeing composition, still more preferably it is a powder hair dyeing composition.

4. The composition according to any of the preceding claims **characterized in that** the total concentration of compound(s) according to groups a) and b) is in the range of 0.05% to 60% by weight, preferably in the range of 0.1% to 50% by weight, more preferably in the range of 0.25% to 40% by weight, still more preferably in the range of 0.3% to 35% by weight, still further more preferably in the range of 0.4% to 35% by weight, calculated to the total weight of composition A.

5. The composition according to any of the preceding claims **characterized in that** the weight ratio of compound(s) according to group a) to compound(s) according to group b) is in the range of 0.2 to 5, preferably in the range of 0.3 to 3, more preferably in the range of 0.5 to 2.

6. The composition according to any of the preceding claims **characterized in that** the total concentration of one or more compound(s) according to group c) is in the range of 0.01% to 20% by weight, preferably in the range of 0.05% to 15% by weight, more preferably in the range of 0.1% to 10% by weight, more preferably in the range of 0.1% to 5% by weight, calculated to the total weight of composition A.

7. The composition according to any of the preceding claims **characterized in that** the weight ratio of compound(s) according to group a) to compound(s) according to group c) is in the range of 0.1 to 20, preferably in the range of 0.25 to 15, more preferably in the range of 1 to 15.

8. The composition according to any of the preceding claims **characterized in that** it comprises one or more direct dye(s) different from group c), and/or their salt(s), and/or their mixtures, preferably it comprises Disperse Black 9, Acid Yellow 1, 2-amino-6-chloro-4-nitrophenol, and/or their salt(s), and/or their mixtures.

9. The composition according to any of the preceding claims **characterized in that** it comprises one or more alkalizing agent(s) as compound(s) according to group d), preferably one or more inorganic alkalizing agent(s), more preferably one or more metal salt of silicate, metasilicate, disilicate, hydroxide, carbonate, bicarbonate, or phosphate, more preferably one or more sodium salt(s) or potassium salt(s) of silicate, metasilicate, disilicate, hydroxide, or phosphate, and/or their mixtures, still more preferably trisodium phosphate or tripotassium phosphate, and/or their mixtures, and preferablythe total concentration of compound(s) according to group d) is in the range 0.1% to 40% by weight, preferably in the range of 0.25% to 30% by weight, more preferably in the range of 0.4% to 25% by weight, still more preferably in the range of 1% to 20% by weight, still further more preferably in the range of 2% to 10% by weight, calculated to the total weight of composition A.

10. The composition according to any of the claims 1 to 2 and 4 to 10 **characterized in that** it is an aqueous composition having a pH in the range of 7 to 12, preferably in the range of 8 to 11.5, further more preferably in the range of 9 to 11.5, further more preferably in the range of 9 to 10.5.

11. The composition according to claim 11 **characterized in that** the total concentration of compound(s) according to groups a) and b) is in the range of 0.01% to 20% by weight, preferably in the range of 0.05% to 15% by weight, more preferably in the range of 0.1% to 12% by weight, still more preferably in the range of 0.25% to 10% by weight, still further more preferably in the range of 0.25% to 8% by weight, calculated to the total weight of composition A.

12. A two-part hair dyeing composition or a three-part hair dyeing and bleaching composition comprising composition A according to any of the preceding claims and an aqueous composition B, preferably having a pH in the range of 1 to 6 and optionally comprising one or more oxidizing agent(s), and optionally a bleaching composition.

13. A kit-of-parts comprising:
- a composition C comprising one or more compound(s) according to groups a) and b) as defined in any of the claims 1 to 11,
- a composition D comprising one or more compound(s) according to group c) as defined in any of the claims 1 to 11,
- an optional aqueous composition B as defined in claim 12.

14. A method for dyeing keratin fibers, preferably human keratin fibers, more preferably human hair, comprising the steps of:
i) providing composition A as defined in any of the claims 1 to 11 and optionally mixing it with composition B as defined in claim 12 to yield a ready-to-use composition having a pH in the range of 7 to 12,
ii) applying the ready-to-use composition onto keratin fibers and leaving it for a time period in the range of 1 min to 60 min,
iii) optionally rinsing-off the keratin fibers and optionally drying the keratin fibers.

15. A method for dyeing keratin fibers, preferably human keratin fibers, more preferably human hair, comprising the steps of:
x) providing composition C as defined in claim 13 and mixing it with composition B as defined in any of the claims 12 and/or 13 to prepare a ready-to-use composition having a pH in the range of 7 to 12,
xi) applying the ready-to-use composition onto keratin fibers and leaving it for a time period in the range of 1 min to 60 min, and optionally rinsing-off the keratin fibers and optionally drying the keratin fibers,
xii) applying composition D as defined in claim 13 onto keratin fibers,
xiii) leaving composition D onto keratin fibers, preferably for a time period in the range of 1 min to 60 min, and optionally rinsing-off the keratin fibers, and optionally drying the keratin fibers.

## Patentansprüche

1. Zusammensetzung A zum Färben von Keratinfasern, vorzugsweise menschlichen Keratinfasern, bevorzugter menschlichem Haar, umfassend:
a) 1,4-Diamino-2-methoxymethylbenzol und/oder dessen Salz(e),
b) einen oder mehreren oxidativen Farbstoffkuppler oder oxidativen Farbstoffvorläufer, die sich von Verbindungen gemäß Gruppe a unterscheiden,
c) ein oder mehrere Direktfarbstoffe, ausgewählt aus HC Blue 18, HC Red 18 und HC Yellow 16, und/oder deren Salz(e) und/oder deren Mischungen.

2. Zusammensetzung nach Anspruch 1, **dadurch gekennzeichnet, dass** eine oder mehrere Verbindung(en) gemäß Gruppe b) 5-Amino-2-methylphenol, 2-Methyl-5-hydroxyethylaminophenol, 2,4,-Diaminophenoxyethanol, 2-Amino-4-hydroxyethylaminoanisol, 2-Methyl-5-amino-6-chlorphenol, 1,3-Bis-(2,4-diaminophenoxy)-propan, 2-Bis(2-hydroxyethyl)-aminotoluol, 2-Amino-5-methylphenol, Resorcin, 2-Methylresorcin, 4-Chlorresorcin, 2-Amino-4-chlorphenol, 5-Amino-4-methoxy-2-methylphenol, 2-Aminophenol, 3-Aminophenol, 1-Methyl-2-hydroxy-4-aminobenzol, 3-N,N-Dimethylaminophenol, 2,6-Dimethyldihydroxy-3,5-dimethylpyridin, 5-Amino-3-methylphenol, 6-Amino-3-methylphenol, 1,3-Diaminobenzol, 1-Amino-3-(2'-hydroxyethylamino)benzol, 1-Amino-3-[bis(2'-hydroxyethyl)amino]benzol, α-Naphtol, 4,6-Dichlorresorcin, 1,3-Diaminotoluol, 4-Hydroxy-1,2-methylendioxybenzol, 1,5-Dihydroxynaphthalin, 1,6-Dihydroxynaphthalin, 1,7-Dihydroxynaphthalin, 2,7-Dihydroxynaphthalin, 1-Hydroxy-2-methylnaphthalin, 4-Hydroxy-1,2-methyldioxybenzol, 2,4-Diamino-3-chlorphenol, 5-Amino-2-methoxyphenol und/oder 1-Methoxy-2-amino-4-(2'-hydroxyethylamino)benzol, 2,4,5,6-Tetraaminopyrimidin, 2,5,6-Triamino-5-pyrimidinol und/oder deren Salz(e) und/oder deren Mischungen, wobei vorzugsweise eine oder mehrere Verbindung(en) gemäß Gruppe b) aus 4-Chlorresorcin, 2-Methylresorcin, 1,3-Bis(2,4-diaminophenoxy)-propan, 2-Methyl-5-amino-6-chlorphenol, 2-Amino-3-hydroxypyridin und/oder deren Salz(e) und/oder deren Mischungen ausgewählt ist/sind.

3. Zusammensetzung nach einem der vorstehenden Ansprüche, **dadurch gekennzeichnet, dass** die Zusammensetzung A eine feste Zusammensetzung, vorzugsweise eine Pulverzusammensetzung oder eine Pelletzusammensetzung oder eine Tablettenzusammensetzung oder eine Kapselzusammensetzung, bevorzugter eine Pulver-Haarfärbezusammensetzung oder eine Pellet-Haarfärbezusammensetzung oder eine Tabletten-Haarfärbezusammensetzung, noch bevorzugter eine Pulver-Haarfärbezusammensetzung ist.

4. Zusammensetzung nach einem der vorstehenden Ansprüche, **dadurch gekennzeichnet, dass** die Gesamtkonzentration der Verbindung(en) gemäß der Gruppen a) und b) im Bereich von 0,05 Gew.-% bis 60 Gew.-%, vorzugsweise im Bereich von 0,1 Gew.-% bis 50 Gew.-%, bevorzugter im Bereich von 0,25 Gew.-% bis 40 Gew.-%, noch bevorzugter im Bereich von 0,3 Gew.-% bis 35 Gew.-%, immer noch bevorzugter im Bereich von 0,4 Gew.-% bis 35 Gew.-%, berechnet auf das Gesamtgewicht der Zusammensetzung A, liegt.

5. Zusammensetzung nach einem der vorstehenden Ansprüche, **dadurch gekennzeichnet, dass** das Gewichtsverhältnis der Verbindung(en) gemäß Gruppe a) zu Verbindung(en) gemäß Gruppe b) im Bereich von 0,2 bis 5, vorzugsweise im Bereich von 0,3 bis 3, bevorzugter im Bereich von 0,5 bis 2, liegt.

6. Zusammensetzung nach einem der vorstehenden Ansprüche, **dadurch gekennzeichnet, dass** die Gesamtkonzentration einer oder mehrerer Verbindung(en) gemäß Gruppe c) im Bereich von 0,01 Gew.-% bis 20 Gew.-%, vorzugsweise im Bereich von 0,05 Gew.-% bis 15 Gew.-%, bevorzugter im Bereich von 0,1 Gew.-% bis 10 Gew.-%, bevorzugter im Bereich von 0,1 Gew.-% bis 5 Gew.-%, berechnet auf das Gesamtgewicht der Zusammensetzung A, liegt.

7. Zusammensetzung nach einem der vorstehenden Ansprüche, **dadurch gekennzeichnet, dass** das Gewichtsverhältnis der Verbindung(en) gemäß Gruppe a) zu Verbindung(en) gemäß Gruppe c) im Bereich von 0,1 bis 20, vorzugsweise im Bereich von 0,25 bis 15, bevorzugter im Bereich von 1 bis 15, liegt.

8. Zusammensetzung nach einem der vorstehenden Ansprüche, **dadurch gekennzeichnet, dass** sie einen oder mehrere Direktfarbstoffe, die sich von der Gruppe c unterscheiden, und/oder deren Salz(e) und/oder deren Mischungen umfasst, wobei sie vorzugsweise Disperse Black 9, Acid Yellow 1, 2-Amino-6-chlor-4-nitrophenol und/oder deren Salz(e) und/oder deren Mischungen umfasst.

9. Zusammensetzung nach einem der vorstehenden Ansprüche, **dadurch gekennzeichnet, dass** sie ein oder mehrere Alkalisierungsmittel als Verbindung(en) gemäß Gruppe d), vorzugsweise ein oder mehrere anorganische Alkalisierungsmittel, bevorzugter ein oder mehrere Metallsalze aus Silikat, Metasilikat, Disilikat, Hydroxid, Carbonat, Bicarbonat oder Phosphat, bevorzugter ein oder mehrere Natriumsalz(e) oder Kaliumsalz(e) von Silikat, Metasilikat, Disilikat, Hydroxid oder Phosphat und/oder deren Mischungen, noch bevorzugter Trinatriumphosphat oder Trikaliumphosphat und/oder deren Mischungen umfasst, und wobei vorzugsweise die Gesamtkonzentration der Verbindung(en) gemäß Gruppe d) im Bereich von 0,1 Gew.-% bis 40 Gew.-%, vorzugsweise im Bereich von 0,25 Gew.-% bis 30 Gew.-%, bevorzugter im Bereich von 0,4 Gew.-% bis 25 Gew.-%, noch bevorzugter im Bereich von 1 Gew.-% bis 20 Gew.-%, immer noch bevorzugter im Bereich von 2 Gew.-% bis 10 Gew.-%, berechnet auf das Gesamtgewicht der Zusammensetzung A, liegt.

10. Zusammensetzung nach einem der Ansprüche 1 bis 2 und 4 bis 10, **dadurch gekennzeichnet, dass** es sich um eine wässrige Zusammensetzung mit einem pH-Wert im Bereich von 7 bis 12, vorzugsweise im Bereich von 8 bis 11,5, bevorzugter im Bereich von 9 bis 11,5, bevorzugter im Bereich von 9 bis 10,5, handelt.

11. Zusammensetzung nach Anspruch 11, **dadurch gekennzeichnet, dass** die Gesamtkonzentration der Verbindung(en) gemäß den Gruppen a) und b) im Bereich von 0,01 Gew.-% bis 20 Gew.-%, vorzugsweise im Bereich von 0,05 Gew.-% bis 15 Gew.-%, bevorzugter im Bereich von 0,1 Gew.-% bis 12 Gew.-%, noch bevorzugter im Bereich von 0,25 Gew.-% bis 10 Gew.-%, immer noch bevorzugter im Bereich von 0,25 Gew.-% bis 8 Gew.-%, berechnet auf das Gesamtgewicht der Zusammensetzung A, liegt.

12. Zweiteilige Haarfärbezusammensetzung oder dreiteilige Haarfärbe- und Bleichzusammensetzung, umfassend Zusammensetzung A nach einem der vorstehenden Ansprüche und eine wässrige Zusammensetzung B, die vorzugsweise einen pH-Wert im Bereich von 1 bis 6 aufweist und optional ein oder mehrere Oxidationsmittel umfasst, und optional eine Bleichzusammensetzung.

13. Teilekit, das Folgendes umfasst:
- eine Zusammensetzung C, die eine oder mehrere Verbindung(en) gemäß den Gruppen a) und b) nach einem der Ansprüche 1 bis 11 umfasst,
- eine Zusammensetzung D, die eine oder mehrere Verbindung(en) gemäß Gruppe c) nach einem der Ansprüche 1 bis 11 umfasst,
- eine optionale wässrige Zusammensetzung B nach Anspruch 12.

14. Verfahren zum Färben von Keratinfasern, vorzugsweise menschlichen Keratinfasern, bevorzugter menschlichem Haar, umfassend die folgenden Schritte:
i) Bereitstellen der Zusammensetzung A nach einem der Ansprüche 1 bis 11 und optionales Mischen mit der Zusammensetzung B nach Anspruch 12, um eine gebrauchsfertige Zusammensetzung mit einem pH-Wert im Bereich von 7 bis 12 zu erhalten,
ii) Auftragen der gebrauchsfertigen Zusammensetzung auf Keratinfasern und Einwirkenlassen für einen Zeitraum im Bereich von 1 min bis 60 min,
iii) optionales Abspülen der Keratinfasern und optionales Trocknen der Keratinfasern.

15. Verfahren zum Färben von Keratinfasern, vorzugsweise menschlichen Keratinfasern, bevorzugter menschlichem Haar, umfassend die folgenden Schritte:
x) Bereitstellen der Zusammensetzung C nach Anspruch 13 und Mischen mit der Zusammensetzung B nach einem der Ansprüche 12 und/oder 13, um eine gebrauchsfertige Zusammensetzung mit einem pH-Wert im Bereich von 7 bis 12 herzustellen,
xi) Auftragen der gebrauchsfertigen Zusammensetzung auf den Keratinfasern und Einwirkenlassen für einen Zeitraum im Bereich von 1 min bis 60 min, und optionales Abspülen der Keratinfasern und optionales Trocknen der Keratinfasern,
xii) Auftragen der Zusammensetzung D nach Anspruch 13 auf den Keratinfasern,
xiii) Einwirkenlassen der Zusammensetzung D auf den Keratinfasern, vorzugsweise für einen Zeitraum im Bereich von 1 min bis 60 min, und optionales Abspülen der Keratinfasern, und optionales Trocknen der Keratinfasern.

## Revendications

1. Composition A de coloration de fibres de kératine, de préférence de fibres de kératine humaines, de manière davantage préférée de cheveux humains, comprenant :
a) du 1,4-diamino-2-méthoxyméthyl-benzène et/ou son ou ses sels,
b) un ou plusieurs coupleurs de coloration oxydatifs ou précurseurs de coloration oxydatifs différents des composés selon le groupe a),
c) un ou plusieurs colorants directs sélectionnés parmi du bleu HC 18, du rouge HC 18 et du jaune HC 16, et/ou leur(s) sel(s), et/ou leurs mélanges.

2. Composition selon la revendication 1, **caractérisée en ce qu'**un ou plusieurs composés selon le groupe b) sont 5-amino-2-méthylphénol, 2-méthyl-5-hydroxyéthylaminophénol, 2,4,-diaminophénoxyéthanol, 2-amino-4-hydroxyéthylaminoanisol, 2-méthyl-5-amino-6-chlorphénol, 1,3-bis-(2,4-diaminophénoxy)-propane, 2-bis(2-hydroxyéthyl)-aminotoluène, 2-amino-5-méthylphénol, résorcinol, 2-méthylrésorcinol, 4-chlororésorcinol, 2-amino-4-chlorophénol, 5-amino-4-méthoxy-2-méthylphénol, 2-aminophénol, 3-aminophénol, 1-méthyl-2-hydroxy-4-aminobenzène, 3-N,N-diméthylaminophénol, 2,6-dihydroxy-3,5-diméthoxypyridine, 5-amino-3-méthylphénol, 6-amino-3-méthylphénol, 1,3-diamino-benzène, 1-amino-3-(2'-hydroxyéthylamino)benzène 1-amino-3-[bis(2'-hydroxy-éthyl)amino]benzène, α-naphthol, 4,6-dichlororésorcinol, 1,3-diamino-toluène, 4-hydroxy-1,2-méthylènedioxybenzène, 1,5-dihydroxynaphtalène, 1,6-dihydroxynaphtalène, 1,7-dihydroxynaphtalène, 2,7-dihydroxynaphtalène, 1-hydroxy-2-méthylnaphtalène, 4-hydroxy-1,2-méthyldioxybenzène, 2,4-diamino-3-chlorophénol, 5-amino-2-méthoxyphénol et/ou 1-méthoxy-2-amino-4-(2'-hydroxyéthylamino)-benzène, 2,4,5,6-tétraaminopyrimidine 2,5,6-triamino-5-pyrimidinol, et/ou leur(s) sel(s), et/ou leurs mélanges, de préférence, un ou plusieurs composés selon le groupe b) est/sont sélectionnés parmi le 4-chlororésorcinol, le 2-méthylrésorcinol, le 1,3-bis(2,4-diaminophénoxy)-propane, le 2-méthyl-5-amino-6-chlorphénol, la 2-amino-3-hydroxypyridine, et/ou leur(s) sel(s), et/ou leurs mélanges.

3. Composition selon l'une quelconque des revendications précédentes **caractérisée en ce que** la composition A est une composition solide, de préférence une composition en poudre ou une composition en pastilles ou une composition en comprimés ou une composition en capsules, de manière davantage préférée une composition de teinture capillaire en poudre ou une composition de teinture capillaire en pastilles ou une composition de teinture capillaire en comprimés, de manière encore davantage préférée une composition de teinture capillaire en poudre.

4. Composition selon l'une quelconque des revendications précédentes **caractérisée en ce que** la concentration totale du ou des composés selon les groupes a) et b) est dans la plage de 0,05 % à 60 % en poids, de préférence dans la plage de 0,1 % à 50 % en poids, de manière davantage préférée dans la plage de 0,25 % à 40 % en poids, de manière encore davantage préférée dans la plage de 0,3 % à 35 % en poids, de manière encore davantage préférée dans la plage de 0,4 % à 35 % en poids, calculée par rapport au poids total de la composition A.

5. Composition selon l'une quelconque des revendications précédentes **caractérisée en ce que** le rapport pondéral entre le(s) composé(s) selon le groupe a) et le(s) composé(s) selon le groupe b) est dans la plage de 0,2 à 5, de préférence dans la plage de 0,3 à 3, de manière davantage préférée dans la plage de 0,5 à 2.

6. Composition selon l'une quelconque des revendications précédentes **caractérisée en ce que** la concentration totale du ou des composés selon le groupe c) est dans la plage de 0,01 % à 20 % en poids, de préférence dans la plage de 0,05 % à 15 % en poids, de manière davantage préférée dans la plage de 0,1 % à 10 % en poids, de manière davantage préférée dans la plage de 0,1 % à 5 % en poids, calculée par rapport au poids total de la composition A.

7. Composition selon l'une quelconque des revendications précédentes **caractérisée en ce que** le rapport pondéral entre le(s) composé(s) selon le groupe a) et le(s) composé(s) selon le groupe c) est dans la plage de 0,1 à 20, de préférence dans la plage de 0,25 à 15, de manière davantage préférée dans la plage de 1 à 15.

8. Composition selon l'une quelconque des revendications précédentes **caractérisée en ce qu'**elle comprend un ou plusieurs colorants directs différents du groupe c), et/ou leur(s) sel(s), et/ou leurs mélanges, de préférence **en ce qu'**elle comprend du noir dispersé 9, du jaune acide 1, 2-amino-6-chloro-4-nitrophénol, et/ou leur(s) sel(s), et/ou leurs mélanges.

9. Composition selon l'une quelconque des revendications précédentes **caractérisée en ce qu'**elle comprend un ou plusieurs agents alcalinisants en tant que composé(s) selon le groupe d), de préférence un ou plusieurs agents alcalinisants inorganiques, de manière davantage préférée un ou plusieurs sels métalliques de silicate, de métasilicate, de disilicate, d'hydroxyde, de carbonate, de bicarbonate ou de phosphate, de manière davantage préférée un ou plusieurs sels de sodium ou sels de potassium de silicate, de métasilicate, de disilicate, d'hydroxyde ou de phosphate, et/ou leurs mélanges, de manière encore davantage préférée du phosphate trisodique ou du phosphate tripotassique, et/ou leurs mélanges, et de préférence la concentration totale du ou des composés selon le groupe d) est dans la plage de 0,1 % à 40 % en poids, de préférence dans la plage de 0,25 % à 30 % en poids, de manière davantage préférée dans la plage de 0,4 % à 25 % en poids, de manière encore davantage préférée dans la plage de 1 % à 20 % en poids, de manière encore davantage préférée dans la plage de 2 % à 10 % en poids, calculée par rapport au poids total de la composition A.

10. Composition selon l'une quelconque des revendications 1 à 2 et 4 à 10, **caractérisée en ce qu'**il s'agit d'une composition aqueuse présentant un pH dans la plage de 7 à 12, de préférence dans la plage de 8 à 11,5, de manière davantage préférée dans la plage de 9 à 11,5, de manière encore davantage préférée dans la plage de 9 à 10,5.

11. Composition selon la revendication 11, **caractérisée en ce que** la concentration totale du ou des composés selon les groupes a) et b) est dans la plage de 0,01 % à 20 % en poids, de préférence dans la plage de 0,05 % à 15 % en poids, de manière davantage préférée dans la plage de 0,1 % à 12 % en poids, de manière encore davantage préférée dans la plage de 0,25 % à 10 % en poids, de manière encore davantage préférée dans la plage de 0,25 % à 8 % en poids, calculée par rapport au poids total de la composition A.

12. Composition de teinture capillaire en deux parties ou composition de teinture capillaire et de décoloration en trois parties comprenant la composition A selon l'une quelconque des revendications précédentes et une composition aqueuse B, de préférence présentant un pH dans la plage de 1 à 6 et comprenant facultativement un ou plusieurs agents oxydants, et facultativement une composition de décoloration.

13. Trousse de pièces comprenant :
- une composition C comprenant un ou plusieurs composés selon les groupes a) et b) telle que définie dans l'une quelconque des revendications 1 à 11,
- une composition D comprenant un ou plusieurs composés selon le groupe c) telle que définie dans l'une quelconque des revendications 1 à 11,
- une composition aqueuse B facultative telle que définie dans la revendication 12.

14. Procédé de coloration de fibres de kératine, de préférence de fibres de kératine humaines, de manière davantage préférée de cheveux humains, comprenant les étapes suivantes :
i) la fourniture de la composition A telle que définie dans l'une quelconque des revendications 1 à 11 et son mélange facultatif avec une composition B telle que définie dans la revendication 12 pour obtenir une composition prête à l'emploi présentant un pH dans la plage de 7 à 12,
ii) l'application de la composition prête à l'emploi sur les fibres de kératine et le fait de la laisser pendant une durée dans la plage de 1 min à 60 min,
iii) le rinçage facultatif des fibres de kératine et le séchage facultatif des fibres de kératine.

15. Procédé de coloration de fibres de kératine, de préférence de fibres de kératine humaines, de manière davantage préférée de cheveux humains, comprenant les étapes suivantes :
x) la fourniture de la composition C telle que définie dans la revendication 13 et son mélange avec la composition B telle que définie dans l'une quelconque des revendications 12 et/ou 13 pour préparer une composition prête à l'emploi présentant un pH dans la plage de 7 à 12,
xi) l'application de la composition prête à l'emploi sur les fibres de kératine et le fait de la laisser pendant une durée dans la plage de 1 min à 60 min, et le rinçage facultatif des fibres de kératine et le séchage facultatif des fibres de kératine,
xii) l'application de la composition D telle que définie dans la revendication 13 sur les fibres de kératine,
xiii) le fait de laisser la composition D sur les fibres de kératine, de préférence pendant une durée dans la plage de 1 min à 60 min, et le rinçage facultatif des fibres de kératine, et le séchage facultatif des fibres de kératine.
